# EUROPÄISCHE PATENTANMELDUNG

(11) **EP 1 277 732 A2**
(43) Veröffentlichungstag der Anmeldung: **22.01.2003**
(21) Anmeldenummer: 02014925.8
(22) Anmeldetag: 08.07.2002
(51) Int. Cl.: C07C 319/24, C07C 321/22

(54) **Verfahren zur Herstellung von polymeren Schwefelverbindungen**

(30) Priorität: 20.07.2001 DE 10134686
(71) Anmelder: BAYER AG, 51368 Leverkusen (DE)
(72) Erfinder: Hahn, Josef, Prof. Dr., 50668 Köln (DE); Runk, Marco, 50321 Brühl (DE); Weidenhaupt, Hermann-Josef, Dr., 50259 Pulheim (DE); Buding, Hartmuth, Dr., 52445 Titz (DE)

(57) **Zusammenfassung**

Die Erfindung betrifft ein einfaches Verfahren zur Herstellung von polymeren Schwefelverbindungen mit Polythiocyclopentandiyl-Struktureinheiten, die als Vulkanisationsmittel für Dienkautschuke dienen.

## Beschreibung

Die Erfindung betrifft ein Verfahren zur Herstellung von polymeren Schwefelverbindungen mit Polythiocyclopentandiyl-Struktureinheiten, die als Vulkanisationsmittel für Dienkautschuke dienen.

Die EP-A 258 168 offenbart die Umsetzung von Olefinen mit Schwefel in Wasser, wobei Basen als Katalysatoren anwesend sein können. Als bevorzugte Olefine werden u.a. Cyclopentadien und Dicyclopentadien genannt (Seite 4, Zeilen 14-15). In den erfindungsgemäßen Beispielen kommt Cyclopentadien defacto nicht vor. Zur industriellen Anwendbarkeit wird auf Seite 7, Zeilen 57-58, und Seite 8, Zeilen 1-3, offenbart, dass die erfindungsgemäßen Vulkanisiermittel zu Vulkanisaten führen, die in ihren physikalischen Eigenschaften vergleichbar sind mit den Eigenschaften, die mit einem konventionellen Schwefelvulkanisiersystem erhalten werden. Eine verbesserte Reversionsstabilität der erfindungsgemäßen Vulkanisate ist nicht beschrieben. Im Beispiel 1 der deutschen Patentanmeldung mit der Anmeldenummer DE 100002878.0 wird gezeigt, dass die dort beschriebenen Produkte aus Dicyclopentadien und Schwefel keine Vulkanisate mit verbesserter Reversionsbeständigkeit ergeben.

Die US-A 3 523 926 offenbart Vulkanisiermittel aus Diolefinen, wie z.B. Cyclopentadien und Dicyclopentadien, und Schwefel mit Aminen als Katalysator. Die zusätzliche Anwendung von Schwefelwasserstoff wird in dieser Schrift weder beschrieben noch nahe gelegt.

Die US-A 2 989 513 offenbart Polymere aus Schwefel und einem Olefin zur Vulkanisation von Kautschuk. In Spalte 3, Zeile 21, wird u.a. Cyclopentadien als nützliches Olefin genannt. Die erfindungsgemäße Reaktion wird bevorzugt zwischen 145° und 160°C durchgeführt. Die Ausführungsbeispiele umfassen nur Copolymere aus Schwefel und Styrol bzw. Schwefel und Ethylen bzw. Isobutylen. An keiner Stelle dieser Schrift wird die zusätzliche Anwendung von Schwefelwasserstoff beschrieben oder nahe gelegt.

In der deutschen Patentanmeldung mit der Anmeldenummer DE 100002878.0 werden polymere Schwefelverbindungen mit Polythiocyclopentandiyl-Struktureinheiten bereits beschrieben, die als Vemetzer für Dienkautschuke eingesetzt werden können.

Nach dieser Patentanmeldung werden die polymeren Schwefelverbindungen erhalten durch Umsetzung der an sich bekannten Di-Cyclopentenylpolysulfane mit Schwefel und Schwefelwasserstoff in Gegenwart von Aminen bei Temperaturen im Bereich von ca. 100° bis 180°C.

Die eingesetzten Di-Cyclopentenylpolysulfane können hergestellt werden durch (a) Addition von Sulfanen an Cyclopentadien und/oder Methylcyclopentadien oder durch (b) Umsetzung der erfindungsgemäßen Cyclopentadiene mit flüssigem Schwefelwasserstoff zu (Methyl)Cyclopent-2-enthiol-(1) und anschließender Umsetzung mit elementarem Schwefel in Gegenwart von Aminen als Katalysator.

Beim Verfahren nach (a) ist die Darstellung und Handhabung der Sulfane aus sicherheitstechnischer Sicht sehr aufwendig, da sich Sulfane beim Kontakt mit rauhen Oberflächen spontan in Schwefelwasserstoff und Schwefel zersetzen können. Beim Verfahren nach (b) ist die Handhabung einer großen Menge an flüssigem Schwefelwasserstoff nötig, wozu es einer Gasverflüssigungsanlage bedarf. Außerdem handelt es sich bei den Verfahren nach (a) und (b) um mehrstufige Verfahren.

Aufgabe der Erfindung war es, ein technisch einfaches und leicht zu realisierendes, einstufiges Verfahren zur Herstellung der polymeren Schwefelverbindungen mit Polythiocyclopentandiyl-Struktureinheiten bereit zu stellen.

Die Aufgabe wurde gelöst durch direkte Umsetzung von (Methyl)Cyclopentadien mit Schwefel und Schwefelwasserstoff in Gegenwart eines Katalysators.

Gegenstand der Erfindung ist daher ein Verfahren zur Herstellung von polymeren Schwefelverbindungen der Formel 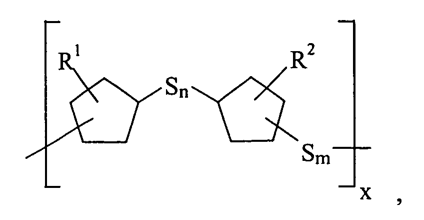 worin die Substituenten
- R¹ und R²: gleich oder verschieden sind und für Wasserstoff oder Methyl stehen,
- n und m: ganze Zahlen im Bereich von 2 bis 12, bevorzugt 2 bis 7,
und
- x: eine ganze Zahl im Bereich von 2 bis 500, bevorzugt 2 bis 300, insbesondere 2 bis 100, bedeuten,
das dadurch gekennzeichnet ist, dass man (Methyl)Cyclopentadien mit Schwefel und Schwefelwasserstoff bei 100° bis 180°C, bevorzugt bei 130° bis 150°C, in Gegenwart eines Katalysators umsetzt, wobei das Molverhältnis von Schwefel zu Schwefelwasserstoff 1:0,1 bis 1:5, bevorzugt 1:0,5 bis 1:2, und das Molverhältnis von (Methyl)Cyclopentadien zu Schwefel 1:1 bis 1:9, bevorzugt 1:2 bis 1:5, beträgt.

Die Zahlen der Schwefelatome n und m in der Polymerkette sind naturgemäß jeweils ganzzahlig. Für die über alle Polymermoleküle gemittelte Schwefelkettenlänge können sich, bedingt durch die Mittelwertbildung, auch gebrochene Zahlen ergeben.

Die Zahl x für die Wiederholungseinheit in einem spezifischen Polymermolekül ist ganzzahlig. Für die mittlere Wiederholungseinheit der Gesamtzahl der Polymeren können sich, bedingt durch die Mittelwertbildung, auch gebrochene Zahlen ergeben.

Als Katalysator für das erfindungsgemäße Verfahren kommen in Frage Brönstedsäuren, Lewissäuren oder Amine. Als Brönstedsäuren werden bevorzugt Phosphorsäure, Perchlorsäure oder Trifluormethansulfonsäure oder auch Mischungen hieraus, eingesetzt. Als Lewissäuren werden bevorzugt wasserfreies Aluminiumchlorid, insbesondere Bortrifluoridetherat, eingesetzt. Als Amine kommen in Frage primäre, sekundäre oder tertiäre, aliphatische, cycloaliphatische oder aromatische oder heterocyclische Amine oder Gemische hieraus. Bevorzugt werden sekundäre oder tertiäre aliphatische Amine mit C₁- bis C₄-Alkylresten eingesetzt, wie z.B. Dimethylamin, Diethylamin, Di-n-propylamin, Di-i-propylamin, Di-n-butylamin, Trimethylamin, Triethylamin, Tri-n-propylamin, Tri-i-propylamin oder Tri-n-butylamin. Besonders bevorzugt ist Triethylamin. Der Katalysator wird im allgemeinen in einer Menge von 0,001 bis 10 Gew.-Teilen, bevorzugt von 0,1 bis 5 Gew.-Teilen, bezogen auf 100 Gew.-Teile Schwefel, eingesetzt.

Die erfindungsgemäße Reaktion findet unter Druck statt, wobei sich dieser in Abhängigkeit von der Art der Einsatzprodukte und deren Menge sowie in Abhängigkeit von der angewendeten Temperatur üblicherweise in einem Bereich von ca. 1 bis 500 bar, bevorzugt ca. 1 bis 250 bar, einstellt.

Das erfindungsgemäße Verfahren kann z.B. nach einem diskontinuierlichen oder kontinuierlichen Verfahren durchgeführt werden. Beim diskontinuierlichen Verfahren wird (Methyl)Cyclopentadien zusammen mit Schwefel, Schwefelwasserstoff und dem Katalysator in einem Druckreaktor vorgelegt und dann das Gemisch unter Rühren auf eine Temperatur von 100° bis 180 °C aufgeheizt und umgesetzt, oder es wird zu der gerührten, auf erfindungsgemäße Temperatur aufgeheizten Mischung (Methyl)Cyclopentadien zugepumpt (Zulaufverfahren). Beim kontinuierlichen Verfahren können die Edukte einzeln oder auch als Gemisch dem Reaktor zugefügt und bei den erfindungsgemäßen Reaktionstemperaturen umgesetzt werden.

In einer besonderen Ausführungsform der Erfindung wird Schwefel, Schwefelwasserstoff und der Katalysator in einem Autoklaven vorgelegt und dann das (Methyl)Cyclopentadien bei der erfindungsgemäßen Reaktionstemperatur zugefügt (Zulaufverfahren).

Die erfindungsgemäße Reaktion kann auch in einem Lösungsmittel durchgeführt werden. Als geeignete Lösungsmittel kommen in Frage aliphatische C₅- bis C₁₂-Kohlenwasserstoffe, wie z.B. Pentan, Hexan, Heptan oder Octan, oder entsprechende Kohlenwasserstoffgemische, wie z.B. Petrolether mit einem Siedepunkt von 40° bis 70°C, Leichtbenzin mit einem Siedepunkt von 70° bis 90°C oder Mittelbenzin mit einem Siedepunkt von 90° bis 180°C, C₅- bis C₁₀-Cycloalkane, wie z.B. Cyclopentan, Methylcyclopentan, Cyclohexan, Methylcyclohexan, Cycloheptan oder Dekalin, oder entsprechende Gemische hieraus, C₁- bis C₅-Halogenalkane, wie z.B. Chlormethane, Fluorchlormethane, Fluorchlorethane oder Tetrachlorethylen, oder entsprechende Gemische hieraus, C₁- bis C₅-Alkohole, wie z.B. Methanol, Ethanol, n- und i-Propanol sowie n-, i- und tert. Butanol, oder entsprechende Gemische hieraus, Ether, wie z.B. Diethylether, Methyl-tert.-Butylether oder Tetrahydrofuran, oder entsprechende Ethergemische hieraus, und (Chlor)Aromaten, wie z.B. Benzol, Toluol, Xylol oder Chlorbenzol, sowie Mischungen hieraus. Selbstverständlich können die genannten Lösungsmittel auch in beliebigen Gemischen untereinander eingesetzt werden. Bevorzugte Lösungsmittel sind Toluol, Methanol, Hexan, Petrolether oder Leichtbenzin. Die Menge an Lösungsmittel beträgt ca. 1 bis 300 Gew.-Teile, bevorzugt ca. 1 bis 150 Gew.-Teile, bezogen auf 100 Gew.-Teile (Methyl)Cyclopentadien.

In einer besonderen Ausführungsform der Erfindung wird ohne Lösungsmittel gearbeitet.

Die Reaktionszeit beträgt etwa 0,5 bis 10 h, bevorzugt etwa 3 bis 6 h. Überschüssiger Schwefelwasserstoff wird aus dem Reaktionsgemisch entfernt und eventuell eingesetztes Lösungsmittel abdestilliert.

### Beispiel (Zulaufverfahren)

Ein mit Stickstoff inertisierter 1,3-1-Rührautoklav wurde mit 139,7 g (4,36 mol) Schwefel, 2,4 g Triethylamin und 180,0 g (5,28 mol) Schwefelwasserstoff beschickt. Unter Rühren wurde der Autoklav auf 140°C geheizt. Bei dieser Temperatur wies der Autoklav einen Innendruck von ca. 70 bar auf. Zu dieser Mischung wurden 96,0 g (1,45 mol) frisch destilliertes Cyclopentadien in ca. 9 Minuten zugepumpt. Nach 4 h Reaktionszeit, gerechnet ab Zulaufende, war der Reaktorinnendruck auf ca. 35 bar abgefallen. Es wurde abgekühlt, entspannt und mit Stickstoff gespült. Als Reaktionsprodukt wurde ein gelber, plastischer Feststoff erhalten, der in Kohlenstoffdisulfid vollständig löslich war. Die Umsetzung verlief praktisch quantitativ.

Es wurden folgende analytische Daten erhalten:

| (C₁₀H₁₆S_{7,5})ₓ (MG:[376,69]ₓ) | | |
|---|---|---|
| C ber.: 31,89% | H ber.: 4,28% | S ber.: 63,83 % |
| C gef.: 31,7% | H gef.: 4,3% | S gef.: 63,8% |

- IR (KBr):: ν = 1437 1/cm (s)
ν = 1313 1/cm (m)
ν = 1240 1/cm (s)
- DSC:: Glastemperatur T_{g} = - 14,3 °C mid point (Aufheizgeschwindigkeit: 5°C/min)
- GPC:: Als Lösemittel für das Reaktionsprodukt wurde ein Gemisch aus Chloroform und Kohlenstoffdisulfid (Volumenverhältnis 10:1) eingesetzt.
Der unlösliche Anteil von ca. 50 Gew.-Teilen, bezogen auf 100 Gew.-Teile an zu lösendem Reaktionsprodukt, wurde abgetrennt.
x = 2 bis 85 (Säule: Jordi Gel DVB, 500 Ä, 500 x 10 mm, Eluent: Chloroform mit 0,5 Gew.-Teilen Ethanol, UV-Detektion: 260 nm, Retentionszeit: 9,7 bis 23 min)
- NMR:: ¹H- und ¹³C-NMR (CDCl₃/CS₂ = 10:1; Volumenverhältnis)

Die typischen Bereiche der chemischen Verschiebung (ppm) bei 1,2-Substitution sind:

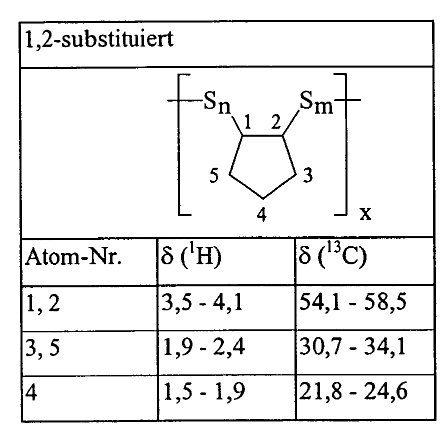

Die typischen Bereiche der chemischen Verschiebung (ppm) bei 1,3-Substitution sind:

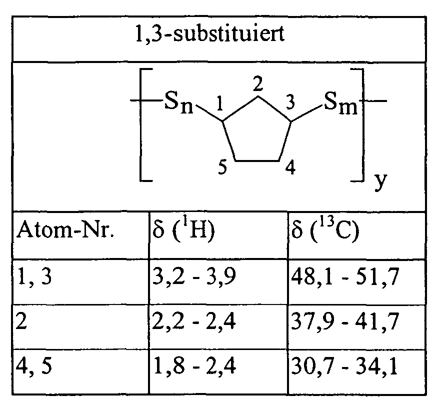

Anteil der 1,3-substituierten Struktur im Polymer: ca. 50 %

## Patentansprüche

1. Verfahren zur Herstellung von polymeren Schwefelverbindungen der Formel 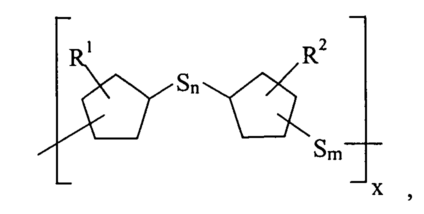 worin die Substituenten
R¹ und R² gleich oder verschieden sind und für Wasserstoff oder Methyl stehen,
n und m ganze Zahlen im Bereich von 2 bis 12
und
x eine ganze Zahl im Bereich von 2 bis 500 bedeuten,
**dadurch gekennzeichnet, dass** man (Methyl)Cyclopentadien mit Schwefel und Schwefelwasserstoff bei 100° bis 180°C in Gegenwart eines Katalysators umsetzt, wobei das Molverhältnis von Schwefel zu Schwefelwasserstoff 1:0,1 bis 1:5 und das Molverhältnis von (Methyl)Cyclopentadien zu Schwefel 1:1 bis 1:9, beträgt.

2. Verfahren nach Anspruch 1, **dadurch gekennzeichnet, dass** als Katalysator Brönstedsäuren, Lewissäuren oder Amine eingesetzt werden.
